# EUROPEAN PATENT APPLICATION

(11) **EP 1 238 626 A2**
(43) Date of publication of application: **11.09.2002**
(21) Application number: 02005044.9
(22) Date of filing: 06.03.2002
(51) Int. Cl.: A61B 5/00, A61B 5/044

(54) **Vital sign display**

(30) Priority: 06.03.2001 JP 2001062437
(71) Applicant: Nihon Kohden Corporation, Shinjuku-ku Tokyo (JP)
(72) Inventor: Kamataki,Osamu, c/oNihon Kohden Corporation, Tokyo (JP); Kawano, Junya, c/oNihon Kohden Corporation, Tokyo (JP); Igarashi, Junichi, c/oNihon Kohden Corporation, Tokyo (JP); Ono, Kohei, c/oNihon Kohden Corporation, Tokyo (JP)
(74) Representative: Grünecker, Kinkeldey, Stockmair & Schwanhäusser Anwaltssozietät

(57) **Abstract**

A bedside monitor includes first display means for simultaneously displaying, in a display area, parameter values of a plurality of vital signs measured for a plurality of patients and displaying alarm information within a parameter display area or an alarm display area in the display area when vital signs relevant to the parameters are in a state where an alarm is to be generated and for displaying prioritized alarm information from among alarm information pertaining to the plurality of vital sign parameters for each patient in the display area; second display means for displaying, in the display area, parameter values of the vital signs and at least a vital sign waveform regarding vital signs of a specified patient among the plurality of patients and alarm information relevant to each of the parameters; and display changeover means, wherein the display area has the function of a touch panel selectable per each display area for each patient when the display are is displayed by the first display means, and the parameter values of the vital signs and the vital sign waveform of a patient selected as a result of pressing the touch panel is displayed by the second display means as the specified patient.

## Description

### BACKGROUND OF INVENTION

### Field of invention

The present invention relates to a vital sign display monitor, and more particularly, to a bedside monitor capable of displaying numerical values and waveforms pertaining to vital signs or setting information, which is input to another bedside monitor connected to the bedside monitor via a network.

### Related Art

In a medical institution, a vital sign display monitor (e.g., a bedside monitor) is disposed at a beside of each bed for monitoring a patient in a bed situated in a ward, a CCU (coronary care unit), an ICU (intensive care unit), or an operating room.

Displayed on a display screen of such a bedside monitor are numerical values or waveforms pertaining to vital signs of a patient in a bed measured by external hardware, or a message pertaining to alarm information.

Individual bedside monitor is connected to a network, and the conditions of patients can be intensively monitored on a vital sign display monitor (central monitor) disposed in a doctor anteroom or a nurse's station.

In recent years, there is a tendency toward a shortage developing in the number of nurses and nursing personnel available. When a nurse is in a patient care visit at midnight or nobody is present at a nurse's station, a doctor or nurse attending a patient at a bedside is required to simultaneously monitor vital signs of other patients in beds.

In order to meet such a demand, a display screen of the bedside monitor connected to the network can also display vital signs of patients in other beds, by means of switching display screens.

However, vital signs to be displayed at one time on a display screen of the bedside monitor connected to the network is limited solely for one patient in a bed except for a patient where the bedside monitor is disposed. Hence, if vital signs of a patient in another bed is to be displayed, a display screen must be switched.

If an attempt is made to monitor a plurality of patients simultaneously, an operation for switching display screens becomes intricate. when an alarm is generated, a current display screen is required to be switched to a display screen for the patient who has caused the alarm and the display screen, for the patient is checked for determination of a factor of the alarm. Moreover, if a plurality of alarms have generated simultaneously, the number of this switching operations increases, and consequently is takes much time to determine factors responsible for the alarms .

### SUMMARY OF THE INVENTION

The present invention has been conceived in light of the foregoing problem and aims at providing a bedside monitor capable of simultaneously displaying, on a display screen, vital signs of a plurality of patients in beds and alarm information by a simple operation; which lessens the burden imposed on a limited number of personnel inmedical care by helping them reduce tasks and shorten working hours; and which enables appropriate and speedy assistance.

To achieve the object, the present invention provides a method of displaying vital signs for use with a bedside monitor, comprising the steps of: simultaneously displaying parameter values of a plurality of sets of vital signs measured for a plurality of patients respectively; and displaying alarm information in a parameter display area or an alarm display area when vital signs relevant to the parameters are in a state where an alarm is to be generated.

As a result, parameter values measured in connection with a plurality of patients can be ascertained simultaneously, and it is possible to ascertain the parameter and patient in an alarm condition.

The present invention also provides a method of displaying vital signs for use with a bedside monitor, comprising the steps of: simultaneously displaying parameter values of a plurality of sets of vital signs measured for a plurality of patients respectively; displaying alarm information in a parameter display area when vital signs relevant to the parameters are in a state where an alarm is to be generated; and displaying prioritized alarm information from among alarm information s pertaining to parameters of the plurality of vital signs in an alarm display area for each patient.

As a result, parameter values measured in connection with a plurality of patients can be ascertained simultaneously, and it is possible to ascertain the parameter and patient in an alarm condition. Further, the most prioritized alarm is displayed and can be ascertained.

The present invention also provides a bedside monitor comprising: first display means for simultaneously displaying parameter values of a plurality of sets of vital signs measured for a plurality of patients respectively in a display area and for displaying alarm information within a parameter display area or an alarm display area in the display area when vital signs relevant to the parameters are in a state where an alarm is to be generated; second display means for displaying parameter values of the vital signs and at least a vital sign waveform regarding vital signs of a specified patient among the plurality of patients in the display area; and display changeover means, the display area having the function of a touch panel selectable per each display area for each patient when the display area is displayed by the first display means, for displaying the parameter values of the vital signs and the vital sign waveform of a patient selected as a result of pressing the touch panel by the second display means as the specified patient.

As a result, by means of a display by the first display means, parameter values measured in connection with a plurality of patients can be ascertained simultaneously, and it is possible to ascertain the parameter and patient in an alarm condition. When further detailed vital signs of a specific patient are desired to be known, detailed vital signs of the patient can be displayed by the second display means by means of the display changeover means.

The present invention also provides a bedside monitor comprising: first display means for simultaneously displaying parameter values of a plurality of sets of vital signs measured for a plurality of patients respectively in a display area and for displaying prioritized alarm information from among alarm information pertaining to the plurality of vital sign parameters for each patient in the display area; second display means for displaying parameter values of the vital signs and at least a vital sign waveform regarding vital signs of a specified patient among the plurality of patients in the display area; and display changeover means, the display area having the function of a touch panel selectable per each display area for each patient when the display area is displayed by the first display means, for displaying the parameter values of the vital sign and the vital sign waveform of a patient selected as a result of pressing the touch panel on the second display means as the specified patient.

As a result, by means of a display by the first display means, parameter values measured in connection with a plurality of patients can be ascertained simultaneously, and the most prioritized alarm displayed and can be ascertained. When detailed vital signs of a specific patient are desired to be known, detailed vital signs of the patient can be displayed by the second display means by means of the display changeover means.

The present invention also provides A bedside monitor comprising: first display means for simultaneously displaying parameter values of a plurality of sets of vital signs measured for a plurality of patients respectively in a display area, when vital signs relevant to the parameters are in a state where an alarm is to be generated, for displaying alarm information in a parameter display area and for displaying prioritized alarm information from among alarm information pertaining to the plurality of vital sign parameters in an alarm display area for each patient; second display means for displaying parameter values of the vital signs and at least a vital sign waveform regarding vital signs of a specified patient among the plurality of patients and alarm information pertaining to the respective parameters in the display area; and display changeover means, the display area having the function of a touch panel selectable per each display area for each patient when the display area is displayed by the first display means, for displaying the parameter values of the vital signs and the vital sign waveform of a patient selected as a result of pressing the touch panel by the second display means as the specified patient.

As a result, by means of a display by the first display means, parameter values measured in connection with a plurality of patients can be ascertained simultaneously, and it is possible to ascertain the parameter and patient in an alarm condition. Moreover, the most prioritized alarm is displayed and can be ascertained. When detailed vital signs of a specific patient are desired to be known, further detailed vital signs of the patient can be displayed by the second display means.

The present invention also provides a vital sign display monitor system comprising: a plurality of bedside monitors described above wherein the plurality of bedside monitors are interconnected via a network, thereby enabling display of vital signs of a plurality of patients.

As a result, a plurality of bedside monitors are disposed in the vicinity of respective patients, and the bedside monitors are connected to a network, whereby vital signs can be shared. Consequently, vital signs of a plurality of patients can be ascertained with a single bedside monitor.

### BRIEF DESCRIPTION OF DRAWING

Fig. 1 is a view showing a display screen of a bedside monitor when tag B "PERSONAL DISP" has been selected;
Fig. 2 is a view showing a display screen of a bedside monitor when tag A "BED DISP" has been selected;
Fig. 3 is a view showing a display screen of a bedside monitor when tag C "SELECTED BED" has been selected; and
Fig. 4 is a view for describing a network to which vital sign display monitors including a central monitor and bedside monitors are connected.

### DETAILED DESCRIPTION OF PREFERRED EMBODIMENTS

An embodiment of a bedside monitor according to the present invention will be described hereinbelow in detail by reference to the accompanying drawings.

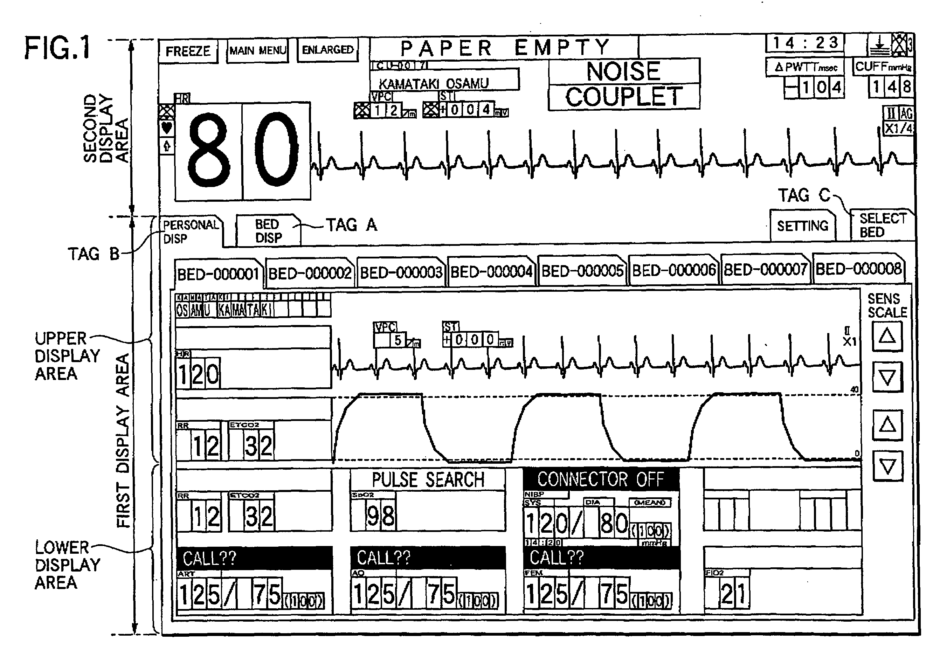

Figs. 1, 2, and 3 are views showing various types of display screens on a bedside monitor.

The area of a display screen is roughly divided into a first display area and a second display area. In various types of display screens, these display areas are fixed.

The first display area includes a bed display (showing vital signs of a plurality of patients in beds), a personal display (displaying vital signs of a selected individual), and bed selection tags (A, B, and C). In accordance with a display mode selected by a tag, various vital signs and alarm information are displayed in the first display area.

At the outset, a display screen for displaying a specified person will be described by reference to Fig. 1. Fig. 1 is a display screen for a case where tag B "PERSONAL DISPLAY" has been selected.

Provided below Tag B are bed number tags (i.e., Bed 1 through Bed 8) to be used for selecting a patient whose vital signs are desired to be displayed by reference to a bed number. By pressing a bed number tag, a current display can be switched to a display of vital signs of an arbitrary patient. When a parameter for a certain patient who is being monitored has changed to an alarm condition, the bed number tag of the patient is highlighted or caused to blink. Thus, a patient in alarm condition is ascertained at first sight. The current display can be switched to a display of the vital signs of a patient in alarm condition, by pressing the bed number tag.

The first display area is divided into an upper display area and a lower display area. In the upper display area, numerical value parameters and relevant vital sign waveforms are displayed in two rows.

Information pertaining to an electrocardiogram, which is one of most important vital signs and needs to be monitored continuously, is fixedly set in an upper row of the upper display area as numerical value parameters and a vital sign waveform relevant thereto. Aheart rate (HR) obtained by detecting R waves in an electrocardiogram is displayed on the left side of the upper row of the upper display area as a numerical value parameter. An electrocardiogram wave form is displayed on the right side of the upper row of the upper display area as the vital sign waveform. Displayed in the vicinity of the electrocardiogram waveform are the number of ventricular premature contractions (VPC) during a predetermined time period and parameters relevant to an ST (an electrocardiogram S-T segment).

Numerical value parameters and a vital sign waveform relevant thereto to be displayed in a lower row of the upper display area are arbitrarily selected and displayed at any time, and can be selected from various parameters displayed in the lower display area.

More specifically, an arbitrary parameter display area in the lower display area has the function of a touch panel. Numerical value parameters and a vital sign such as a vital sign waveform relevant thereto which are to be displayed in a lower row in the upper display area are selected by touching the touch panel with a finger. Here, the lower display area displays a plurality of parameters simultaneously, and hence numerical value parameters are displayed in the lower display area; no waveforms are displayed in the lower display area.

An alarm display area for displaying alarms related to parameters is provided in the numerical value parameter display area. Alarms for all the parameters being displayed can be ascertained simultaneously.

A vital sign waveform to be displayed can be selected from a respiration waveform, a CO₂ waveform, a SpO₂ waveform (a pulse wave waveform), a PRESS waveform (a pressure waveform), and OFF (no waveform).

Displayed as numerical-value parameters of vital signs are a heart rate (HR), a pulse rate (PR), the number of ventricular premature contractions (VPC) during a given time period, an electrocardiogram S-T segment (ST), an electrocardiogram R-R interval (RR), a carbon dioxide partial pressure (CO₂), oxygen saturation in arterial blood (SpO₂), a non-invasive blood pressure (NIBP), a pressure value (PRESS), an inspired oxygen fractional concentration (FiO₂), and a body temperature (TEMP).

Different labels are affixed to pressure values (PRESS) of individual sites in the body. Indication of such a label enables a distinction between sites to be measured. Labels showing the following sites can be selected and used.
- ART:: arterial pressure
- RAD:: radial artery pressure
- DORS:: dorsal artery pressure
- AO:: aortic pressure
- FEM:: femoral artery pressure
- UA:: umbilical artery (UA) pressure
- UV:: umbilical vein (UV) pressure
- PAP:: pulmonary artery pressure (PAP)
- CVP:: central venous pressure (CVP)
- RAP:: right artery pressure (RAP)
- RVP:: right ventricular pressure (RVP)
- PRESS:: Others
- ICP:: intracranial pressure (ICP)

Numerical values and waveforms pertaining to vital signs set forth can be displayed.

Alarm information provided in Table 1 is one type of alarm information pertaining to various vital signs and an alarm message for reporting changes in condition of a patient when a threshold value is exceeded.

Table 1 provides types of vital signs and threshold values (upper limit, lower limit, and upper and lower limits) responsible for an alarm, and a character string of an alarm message to be displayed (highlighting of a measured numerical value or display of a specific character string).

**TABLE 1**

| ALARM FACTORS | | ALARM MESSAGES |
|---|---|---|
| HR (ECG) | UPPER AND LOWER LIMITS | HIGHLIGHTING OF NUMERICAL VALUE |
| | PACING | PACING |
| ST | UPPER AND LOWER LIMITS | HIGHLIGHTING OF NUMERICAL VALUE |
| VPC | UPPER LIMIT | HIGHLIGHTING OF NUMERICAL VALUE |
| PR | UPPER AND LOWER LIMITS | HIGHLIGHTING OF NUMERICAL VALUE |
| PRESS | UPPER AND LOWER LIMITS | HIGHLIGHTING OF NUMERICAL VALUE |
| NIBP | UPPER AND LOWER LIMITS | HIGHLIGHTING OF NUMERICAL VALUE |
| SpO₂ | UPPER AND LOWER LIMITS | HIGHLIGHTING OF NUMERICAL VALUE |
| RR | UPPER AND LOWER LIMITS | HIGHLIGHTING OF NUMERICAL VALUE |
| | APNEA | APNEA |
| TEMP (ΔT) | UPPER AND LOWER LIMITS | HIGHLIGHTING OF NUMERICAL VALUE |
| CO₂ | UPPER AND LOWER LIMITS | HIGHLIGHTING OF NUMERICAL VALUE |
| FiO2 | UPPER AND LOWER LIMITS | HIGHLIGHTING OF NUMERICAL VALUE |

Alarm information pertaining to a specific vital sign includes for instance alarm information pertaining to arrhythmia.

Table 2 provided below provides categories of arrhythmia responsible for an alarm and character strings of alarm messages to be displayed (i.e., display of a specific character string).

**TABLE 2**

| ARRHYTHMIA CAUSING ALARM | ALARM MESSAGES |
|---|---|
| Asystole | ASYSTOLE |
| ventricular fibration | VF |
| ventricular tachycardia | VT |
| Tachycardia | TACHYCARDIA |
| Bradycardia | BRADYCARDIA |
| short-run-type VPCs | VPC RUN |
| couplet = paired VPCs | COUPLET |
| Early VPC | EARLY VPC |
| Ventricular bigeminy | BIGEMINY |
| Frequent VPC | FREQ. VPC |

Categories of arrhythmia provided in Table 2 and conditions for displaying alarm messages will be described below.

ASYSTOLE means asystole. If asystole continoues over a time period longer than a preset time, ASYSTOLE is displayed.

VF means ventricular fibrillation. When ventricular fibrillation has been detected, VF is displayed.

VT means ventricular tachycardia. When ventricular premature contraction (VPC) has occurred continuously nine beats or more, VT is displayed (when the number of heart beats exceeds 100).

TACHYCARDIA means tachycardia. When heart rate exceeds a specified value, TACHYCARDIA is displayed.

BRADYCARDIA means bradycardia. When heart rate is smaller than a specified value, BRADYCARDIA is displayed.

VPC RUN means a short-run-type VCP. When VPC has occurred continuously more than specified times, VPC RUN is displayed.

COUPLET means couplet. When VPC has occurred twice continuously, COUPLET is displayed.

EARLY VPC means early systolic ventricular premature contraction. When VPC has occurred and the R-R interval is one-third of a normal one , EARLYVPC is displayed.

BIGEMINY means ventricular bigeminy. When three or more sets of ventricular bigeminy have occurred, BIGEMINY is displayed.

FREQ. VPC means frequent VPC. When the number of VPCs becomes more than preset numbers in one minute, FREQ. VPC is displayed.

Alarm information pertaining to an environment of an instrument used for measuring the foregoing vital signs including alarm information is provided in Table 3.

**TABLE 3**

| Vital Sign | Cause of alarm | Alarm message |
|---|---|---|
| ECG | Connector off | CONNECTOR OFF |
| | Electrode check | CHECK ELECTRODES |
| | Noise | NOISE |
| | An electrocardiogram being learned | LEARNING |
| PRESS | Connector off | CONNECTOR OFF |
| | Sensor check | CHECK SENSOR |
| | Out of measuring range | OUT OF RANGE |
| | No zero setting | CAL?? |
| | Out of zero range | ZERO OUT OF RANGE |
| NIBP | Connector off | CONNECTOR OFF |
| | Check cuff or hose | AIR LEAK |
| SpO₂ | Connector off | CONNECTOR OFF |
| | Probe check | CHECK PROBE |
| | Detection of a pulse wave is impossible | NO PULSE |
| | Pulse wave being retrieved | PULSE SEARCH |
| RR | Connector off | CONNECTOR OFF |
| TEMP | Connector off | CONNECTOR OFF |
| | Sensor check | CHECK SENSOR |
| CO₂ | Connector off | CONNECTOR OFF |
| | Sensor check | CHECK SENSOR |
| FiO₂ | Connector off | CONNECTOR OFF |
| | Sensor check | CHECK SENSOR |
| | Uncalibrated | CAL?? |

As an alarm pertaining to a bedside monitor itself, there may also be displayed an alarm for warning of occurrence of a glitch or failure in the vital sign display monitor, such as an anomaly in the SpO₂ circuit, an anomaly in an NIBP safety circuit, or a battery drain.

Display colors on a display screen for the foregoing alarm maybe set in compliance with international standards, such as those of IEC, so that the vital sign display monitor can be used throughout the world.

By means of sensitivity scale keys provided beside respective waveforms in the first display area, the sensitivity of a display waveform can be switched.

Vital signs for one patient in a bed are displayed in the second display area. Settings are usually effected so as to monitor vital signs of a patient in a bed where the bedside monitor is situated.

An electrocardiogram (ECG) is primarily displayed as a waveform of a vital sign. The bedside monitor may be set so that a waveform can be selected from a respiration waveform (RESP), a CO₂ waveform (CO₂), a pulse wave waveform (SpO₂), a pressure waveform (PRESS), and no waveform (OFF).

Displayed as numerical-value parameters of vital signs are a heart rate (HR), a pulse rate (PR), the number of ventricular premature contractions (VPC) in a predetermined time period, an electrocardiogram S-T segment (ST), an electrocardiogram R-R interval (RR), a carbon dioxide partial pressure (CO₂), oxygen saturation in an arterial blood (SpO₂), non-invasive blood pressure (NIBP), a pressure value (PRESS), an inspired oxygen fractional concentration (FiO₂), and a body temperature (TEMP).

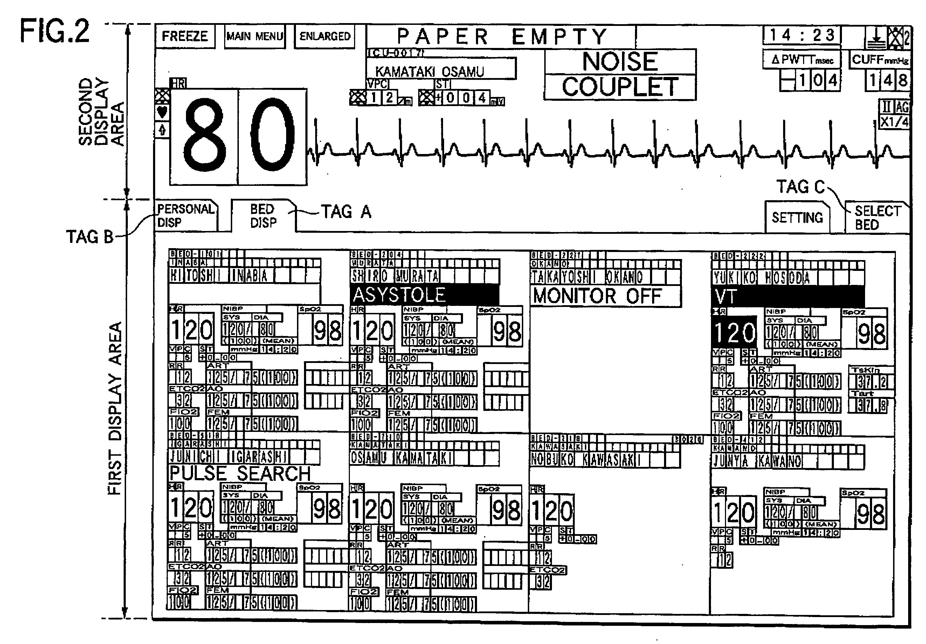

Next, a display screen for displaying vital signs of a plurality of patients in beds will now be described. Fig. 2 shows a display screen for a case where tag A"BED DISP" has been selected.

Here, vital signs and alarm information pertaining to patients in eight selected beds are displayed. Numerical value parameters of vital signs measured from the respective patients are displayed concurrently. In order to indicate an alarm condition of each of the parameters or vital signs relevant thereto, an area in which a numerical value parameter of a vital sign in an alarm condition is displayed in a highlighting or blinking manner (e.g., an HR display area of a bed 222).

In connection with alarm information messages, an alarm considered to be most prioritized to other alarms is displayed in an alarm display area as a result of comprehensively considering the kind of alarm for each vital sign signal, whether or not a threshold value delineating a dangerous range in the numerical value has been exceeded, and a measurement environment alarm or a like factor. If the currently-displayed alarm cannot be ignored, the alarm display area can be visually notified by highlighting or blinking.

Consequently, by means of the first display area, all numerical value parameters which have been measured with regard to vital signs of all the patients in the eight displayed beds can be visually ascertained simultaneously. At that time, a determination can also be made as to whether all the parameters fall within normal ranges or whether they constitute alarm conditions. Moreover, the nature of the most important alarm for each patient and a determination as to whether or not an urgent measure is necessary can also be ascertained.

Each bed display area in the first display area has the function of a touch panel. By touching a display area for any patient in a bed with a finger, the display screen in the first displaying area is switched to the personal display shown in Fig. 1, for the patient.

The display screen shown in Fig. 2 shows a detailed alarm condition for each parameter. Further, if a vital sign waveform or a detailed alarm condition for each parameter of a certain patient is desired to be ascertained, a bed display screen of the patient in the bed selected as shown in Fig. 1 is displayed, by touching a display area for the patient, thereby enabling ascertainment of the detailed alarm condition or the vital sign waveform for each parameter.

The second display area is identical with the second display area shown in Fig. 1.

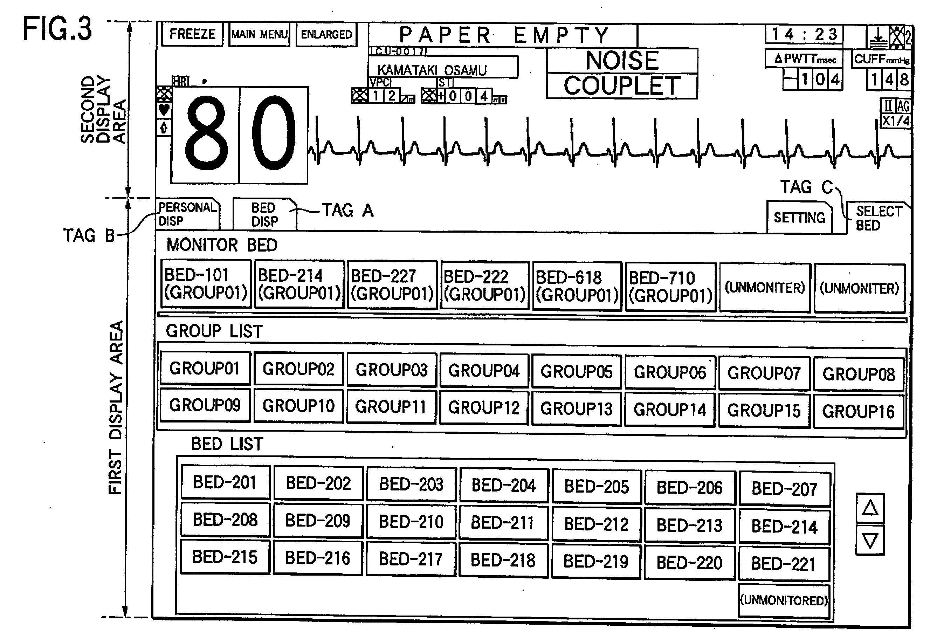

A bed selection display screen will now be described by reference to Fig. 3.

Fig. 3 shows a display screen for a case where tag C "SELECT BED" has been selected.

Displayed in the first display area are keys for selecting a specific bed from all beds for each item (i.e., MONITOR BED keys, GROUP LIST keys, and BED LIST keys) for registering a bed to be displayed on the personal display screen (Fig. 1) or the bed display screen (Fig. 2).

There will now be described a method of registering eight beds to be displayed in the first display area on the bed display screen, provided that all beds have already been classified into groups.

First, a key to be registered is selected from the MONITOR BED keys.

Next, a group to which the bed to be registered belongs is selected from the GROUP LIST keys. A list of selected groups are displayed in the BED LIST keys. The number of keys to be displayed is limited by the width of an area in display screen (21 for the case of the display screen shown in Fig. 3). Hence, if the number of beds is large, keys to be displayed can be switched, by means of a scroll key provided beside the BED LIST keys.

By pressing a specific key from among the BED LIST keys, the bed can be registered into the selected MONITOR BED key.

The thus-determined bed is displayed in the display areas for each "BED" on the bed display screen.

When a bed registered in a bedside monitor is to be deleted, a key to be deleted is selected from the MONITOR BED keys, and "UNMONITORED key" in the BED LIST keys is pressed, thus the bed is deleted.

The second display area is identical with that mentioned previously.

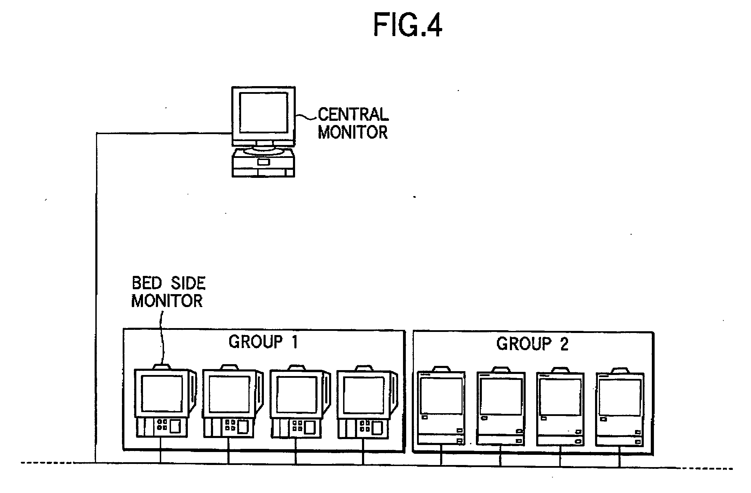

A network to which a plurality of bedside monitors according to the present embodiment are connected will now be described by reference to Fig. 4.

As shown in Fig. 4, bedside monitors provided for respective patients can be grouped into categories; for example, Group 1 and Group 2. The groups can be used; for example, as a group for a ward, a CCU, an ICU, or an operating room.

In order to intensive monitoring, a vital sign display monitor (i.e., central monitor) for management purpose can be provided in a doctor anteroom or a nurse's station.

Bedside monitors assigned to respective groups and the central monitor are connected to a network, such as a LAN, and can effect data communication.

According to the present invention, there is provided a method of displaying vital signs for use with a bedside monitor, comprising the steps of: simultaneously displaying parameter values of a plurality of sets of vital signs measured for a plurality of patients respectively; and displaying alarm information in a parameter display area or an alarm display area when vital signs relevant to the parameters are in a state where an alarm is to be generated. As a result, there can be provided a vital sign display method which enables simultaneous ascertainment of parameter values measured in connection with a plurality of patients and ascertainment of the parameter and patient in an alarm condition.

According to the present invention, there is provided a method of displaying vital signs for use with a bedside monitor comprising the steps of : simultaneously displaying parameter values of a plurality of sets of vital signs measured for a plurality of patients respectively; displaying alarm information in a parameter display area when vital signs relevant to the parameters are in a state where an alarm is to be generated; and displaying prioritized alarm information, from among alarm information pertaining to parameters of the plurality of vital signs, in an alarm display area for each patient. As a result, there can be provided a vital sign display method which enables simultaneous ascertainment of parameter values measured in connection with a plurality of patients, ascertainment of the parameter and patient in an alarm condition, and ascertainment of the most prioritized alarm.

According to the present invention, there is provided a bedside monitor comprising: first display means for simultaneously displaying, in a display area, parameter values of a plurality of sets of vital signs measured for a plurality of patients respectively and displaying alarm information within a parameter display area or an alarm display area in the display area when vital signs relevant to the parameters are in a state where an alarm is to be generated; second display means for displaying, in the display area, parameter values of the vital signs and at least a vital sign waveform regarding vital signs of a specified patient of the plurality of patients; and display changeover means, wherein the display area has the function of a touch panel selectable per each display area for each patient when the display area is displayed by the first display means, and the parameter values of the vital signs and the vital sign waveform of a patient selected as a result of pressing the touch panel is displayed by the second display means as the specified patient. As a result, there can be provided a bedside monitor which enables, by means of a display by the first display means, simultaneous ascertainment of parameter values measured in connection with a plurality of patients, ascertainment of the parameter and patient in an alarm condition, and instantaneous display of further detailed vital signs of the patient by the second display means by means of the display changeover means when further detailed vital signs of a specific patient are desired to be known.

According to the present invention, there is provided a bedside monitor comprising: first display means for simultaneously displaying, in a display area, parameter values of a plurality of sets of vital signs measured for a plurality of patients respectively and displaying, in the display area, prioritized alarm information from among alarm information pertaining to the plurality of vital sign parameters for each patient; second display means for displaying, in the display area, parameter values of the vital signs and at least a vital sign waveform regarding vital signs of a specified patient among the plurality of patients; and display changeover means, wherein the display area has the function of a touch panel selectable per each display area for each patient when the display area is displayed by the first display means, and the parameter values of the vital signs and the vital sign waveform of a patient selected as a result of pressing the touch panel is displayed by the second display means as the specified patient. As a result, there can be provided a bedside monitor which enables, by means of a display by the first display means, simultaneous ascertainment of parameter values measured in connection with a plurality of patients, ascertainment of the most prioritized displayed alarm , and instantaneous display of further detailed vital signs of the patient by the second display means by means of the display changeover means when detailed vital signs of a specific patient are desired to be known.

According to the present invention, there is provided a bedside monitor comprising: first display means for simultaneously displaying, in a display area, parameter values of a plurality of sets of vital signs measured for a plurality of patients respectively and displaying alarm information in a parameter display area, thereby displaying, in an alarm display area and for each patient, prioritized alarm information from among alarm information pertaining to the plurality of vital sign parameters; second display means for displaying, in the display area, parameter values of the vital signs, and at least a vital sign waveform regarding vital signs of a specified patient among the plurality of patients, and alarm information pertaining to the respective parameters; and display changeover means, wherein the display area has the function of a touch panel selectable per each display area for each patient when the display area is displayed by the first display means, and the parameter values of the vital signs and the vital sign waveform of a patient selected through pressing of the touch panel is displayed by the second display means as the specified patient. As a result, there can be provided a bedside monitor which enables, by means of a display by the first display means, simultaneous ascertainment of parameter values measured in connection with a plurality of patients, ascertainment of the parameter and patient in an alarm condition, ascertainment of the most prioritized displayed alarm, and instantaneous display of further detailed vital signs of the patient by the second display means when detailed vital signs of a specific patient are desired to be known.

According to the present invention, there is provided a vital sign display monitor system comprising: a plurality of bedside monitors described above, wherein the plurality of bedside monitors are interconnected via a network, thereby enabling display of vital signs of a plurality of patients. As a result, there can be provided a vital sign display monitor system, in which a plurality of bedside monitors are disposed in the vicinity of respective patients and the bedside monitors are connected to a network, whereby vital signs can be shared, and vital signs of a plurality of patients can be ascertained with a single bedside monitor.

## Claims

1. A method of displaying vital signs for use with a bedside monitor, comprising the steps of:
simultaneously displaying parameter values of a plurality of sets of vital signs measured for a plurality of patients respectively; and
displaying alarm information in a parameter display area or an alarm display area when vital signs relevant to the parameters are in a state where an alarm is to be generated.

2. A method of displaying vital signs for use with a bedside monitor including:
simultaneously displaying parameter values of a plurality of sets of vital signs measured for a plurality of patients respectively;
displaying alarm information in a parameter display area when vital signs relevant to the parameters are in a state where an alarm is to be generated ; and
displaying prioritized alarm information from among alarm information pertaining to parameters of the plurality of vital signs in an alarm display area for each patient.

3. A bedside monitor comprising:
first display means for simultaneously displaying parameter values of a plurality of sets of vital signs measured for a plurality of patients respectively in a display area and for displaying alarm information within a parameter display area or an alarm display area in the display area when vital signs relevant to the parameters are in a state where an alarm is to be generated;
second display means for displaying parameter values of the vital signs and at least a vital sign waveform regarding vital signs of a specified patient among the plurality of patients in the display area; and
display changeover means, the display area having the function of a touch panel selectable per each display area for each patient when the display area is displayed by the first display means, for displaying the parameter values of the vital signs and the vital sign waveform of a patient selected as a result of pressing the touch panel by the second display means as the specified patient.

4. A bedside monitor comprising:
first display means for simultaneously displaying parameter values of a plurality of sets of vital signs measured for a plurality of patients respectively in a display area and for displaying prioritized alarm information from among alarm information pertaining to the plurality of vital sign parameters for each patient in the display area;
second display means for displaying parameter values of the vital signs and at least a vital sign waveform regarding vital signs of a specified patient among the plurality of patients in the display area; and
display changeover means, the display area having the function of a touch panel selectable per each display area for each patient when the display area is displayed by the first display means, for displaying the parameter values of the vital signs and the vital sign waveform of a patient selected as a result of pressing the touch panel by the second display means as the specified patient.

5. A bedside monitor comprising:
first display means for simultaneously displaying parameter values of a plurality of sets of vital signs measured for a plurality of patients respectively in a display area, when vital signs relevant to the parameters are in a state where an alarm is to be generated, for displaying alarm information in a parameter display area and for displaying prioritized alarm information from among alarm information pertaining to the plurality of vital sign parameters in an alarm display area for each patient;
second display means for displaying parameter values of the vital signs and at least a vital sign waveform regarding vital signs of a specified patient among the plurality of patients and alarm information pertaining to the respective parameters in the display area; and
display changeover means, the display area having the function of a touch panel selectable per each display area for each patient when the display area is displayed by the first display means, for displaying the parameter values of the vital signs and the vital sign waveform of a patient selected as a result of pressing the touch panel on the second display means as the specified patient.

6. A vital sign display monitor system comprising:
a plurality of bedside monitors described in any one of claims 3 through 5,
wherein the plurality of bedside monitors are interconnected via a network, thereby enabling display of vital signs of a plurality of patients.
